Europäisches Patentamt

European Patent Office

Office européen des brevets

(11) Publication number:
**0 355 053**
**A2**

## EUROPEAN PATENT APPLICATION

(21) Application number: 89308073.9

(51) Int. Cl.4: **G01N 33/28**

(22) Date of filing: 09.08.89

(30) Priority: 16.08.88 GB 8819459

(43) Date of publication of application:
21.02.90 Bulletin 90/08

(84) Designated Contracting States:
AT BE CH DE ES FR GB GR IT LI LU NL SE

(71) Applicant: EXXON CHEMICAL PATENTS INC.
200 Park Avenue
Florham Park New Jersey 07932(US)

(72) Inventor: Smith, Darryl Royston Terence
27 Denbeck Wood
Eastleaze Swindon Wiltshire(GB)
Inventor: Ibrahim, Tuncel
42 Alexander Close
Abingdon Oxfordshire OX14 1XB(GB)

(74) Representative: Bawden, Peter Charles et al
EXXON CHEMICAL LIMITED EXXON
CHEMICAL TECHNOLOGY CENTRE PO Box 1
Abingdon Oxfordshire OX13 6BB(GB)

(54) Process for assessing wax settling in a fuel.

(57) The amount of settling of crystallised wax in a fuel e.g. diesel fuel at low temperatures may be more quickly assessed by cooling the fuel more rapidly, by centrifuging the cooled fuel and observing the volume of settled cloudy material. Results obtained in a few hours correlate well with results obtained using a three to five day test which simulates conditions of crystal formation and settling in a fuel tank.

EP 0 355 053 A2

# PROCESS FOR ASSESSING WAX SETTLING IN A FUEL

The present invention relates to a process for assessing the amount of settling of wax in a fuel, particularly a distillate petroleum fuel at low temperature.

Crude and refined petroleum fuels e.g. diesel fuel and heating oil, contain ranges of n-alkanes that separate out as solid wax below the fuel cloud point. For example when a $0°C$ cloud point diesel fuel is kept at below $0°C$ for an extended period of time e.g. during a cold spell in winter, the wax crystallizes out into plate-like crystals that gel the fuel and prevent its passage through narrow pipes and filters. To lower the temperature at which wax crystallization limits the use and distribution of such fuels, certain wax crystal modifying additives are added to reduce the size and alter the shapes of wax crystals formed. For example, it is common practice to add low molecular weight copolymers of ethylene and unsaturated esters, in particular ethylene vinyl acetate (EVAC) copolymers, to diesel fuels and heating oils. Using such additives formation of plate-like crystals of about 0.5 to 5 mm in size may be reduced to formation of needle-like crystals of 20 to 50 $\mu$m in length. As a result of using such EVAC additives, the gel no longer forms and the fuel with wax may still be used several degrees below its cloud point. The fuel will still flow and most diesel vehicles will continue to operate down to a lower temperature than would be possible without the additives.

To assess the quality of a fuel, and in particular its performance at low temperature, a test is carried out to measure the temperature at which the crystallized wax blocks or plugs a filter. This temperature is known as the cold filter plugging point (CFPP). Many countries set standards for CFPP which must be met oils and fuels. The test is carried out by pumping the oil or fuel through a filter and observing the temperature at which the filter becomes blocked by wax.

Whilst this test assesses the continuous flow properties of the fuel, it does not provide any information about the settling properties of the wax crystals.

Prolonged storage at sub-cloud temperatures e.g. $10°C$ below the cloud point will result not only in wax crystallization but, if the fuel remains static for e.g. several days at this temperature, then the wax crystals will settle and agglomerate at the base of the fuel storage tank. A practical result of this phenomenon is that some diesel vehicles will show premature failures at temperatures above the fail temperature in the absence of wax settling. Also, fuel in exposed storage tanks will become inhomogeneous, delivering wax enriched fuel at times (with related cold flow problems) and wax depleted fuel at other times. To alleviate this problem a wax anti-settling agent (WASA) such as is described in EP-B-0030099, may be added to the fuel.

As indicated above, the CFPP test does not take into account wax settling. Above the CFPP most diesel vehicles will have no operability problems if there is no wax settling. However, if wax settling occurs, this causes problems i.e. engine failure at temperatures above the CFPP. The CFPP is thus inadequate since two fuels which have the same cold filter plugging point will not necessarily show the same tendency for the wax crystals to settle.

A test as described in EP-B-0030099 was therefore devised to assess the amount of settling of wax in a fuel; this assessment method simulated the conditions in a fuel tank. A sample of the fuel in a graduated flask was gradually cooled e.g. at the rate of $1°C$/hour to the test temperature to ensure that the size and shape of crystals formed corresponded with the size and shape of crystals formed in practice. Once the test temperature, generally 5 to $15°C$ below the cloud point of fuel was reached, the fuel was held at that temperature for three to five days allowing the crystals to settle under gravity in the same way they would in a fuel tank.

However, this test is time consuming and does not allow rapid assessment of the wax settling properties of a fuel. As such, it cannot be used at a refinery, where an assessment would be required fairly rapidly. It has now been found that a fuel may be centrifuged in a refrigerated centrifuge, to deliberately cause the wax crystals to settle, and that the volume of settled cloudy material correlates well with the amount of settled material obtained using the 3-5 day test. It is surprising that this centrifugation of a fuel containing asymmetrical crystals, such as the needle-shaped wax crystals, results in the crystals settling in substantially the same way as during the three to five day test under gravity.

Thus the present invention provides a process for assessing the amount of settling of wax in a fuel at a temperature less than the fuel cloud point comprising centrifuging the fuel at the temperature and observing the volume of settled material.

The appearance of the fuel e.g. a wax settled layer with clear or cloudy supernatant or homogeneous cloudy fuel, may be observed. Further information on the wax-settling properties may be obtained by observing the fuel after the test as it warms up.

The test temperature is generally from 5 to $15°C$ below cloud point of fuel.

The cloud point of a fuel and the temperature at which the fuel is expected to function depends on the climate in which the fuel is used. For example in North-West Europe the temperature at which diesel fuels are required to perform is generally -10 to -15° C; in Scandanavia it is -25° C and in India it may only be +10° C.

The centrifuge speed used in the test is not usually critical; the faster the speed the more rapid the settling, and therefore the quicker the test is completed. However, generally a centrifuge speed of more than 2500 r.p.m. tends to cause all the wax crystals to settle, even those which were initially present as a dispersion and which would not settle over a period of 3-5 days. It is therefore preferred to use a speed of less than 2500 r.p.m.

Generally the centrifugation is carried out for a period of 35 minutes to 8 hours, depending on the centrifuge speed. Typically at a speed of e.g. 500 r.p.m. the samples are centrifuged for about an hour.

It has been found that the test may be further speeded up by cooling the fuel from room temperature to the test temperature in a time of from 45 minutes to 8 hours; typically 100ml of fuel may be brought to a temperature of -10 to 15° C within 1 hour. Although this more rapid cooling of the fuel will produced wax crystals which are smaller than those produced in practice in a fuel tank, it has been found that if the more rapidly cooled fuel is centrifuged, the results in terms of volume of cloudy settled material which is observed surprisingly correlate well with the results obtained using the 3-5 day test in which the conditions of crystal formation and settling simulate those which occur in practice.

The present method may be used both to assess the amount of wax which settles in a fuel, and also to assess a suitable amount of wax anti-settling agent needed to be added to a fuel. To carry out this latter assessment a plurality of tests as described above are carried out, each test using a different amount of wax anti-settling agent and comparing the results. Commercial centrifuges are generally capable of holding 2 to 20 samples of fuel, enabling a series of tests to be carried out simultaneously using different amounts of wax anti-settling agent. The minimum amount of WASA necessary to produce a sufficient anti-settling effect can then be ascertained. In general if the volume of cloudy fuel is 70% or more and settled material is 10% or less there is considered to be sufficient wax anti-settling agent present.

The following examples illustrate the invention.

## EXAMPLE 1

Samples of the fuel were placed in 100ml graduated centrifuged tubes. The samples either contained ethylene vinyl acetate (EVAC) or a wax anti-settling agent (WASA).

Over a period of an hour samples are brought to a temperature of -12° C and equilibrated at that temperature. They are then centrifuged at 500 r.p.m. for 1 hour. The volume which is clear or cloudy in each sample after centrifugation is shown in table 1 column (a). Table 1 also indicates in column (b) the results of the three day test for assessing the amount of wax settling when carried out on the same fuel with the same quantity of additive. It can be seen that centrifuging the fuel for 1 hour at 500 r.p.m. produces settling which correlates well with the settling obtained in the three day test.

TABLE 1

|  | ADDITIVE | (a) | (b) |
|---|---|---|---|
| Temp (° C)<br>Time (hour)<br>Speed (rpm) |  | -12<br>1<br>500 |  |
| FUEL |  |  |  |
| Tank 198 | EVAC<br>WASA | 25% Clear<br>7% Cloudy | 20% Clear<br>5% Cloudy |
| Tank 197 | EVA<br>WASA | 6% Clear<br>3% Cloudy | 6% Clear<br>No wax Settling |
| (a) results using CENTRIFUGE<br>(b) results at -10° C for 3 DAYS under gravity | | | |

Details of the fuels used in this and the following examples are given in Table 2.

3

TABLE 2

| D-86 DISTILLATION (DEG C) | | | | | | | |
|---|---|---|---|---|---|---|---|
| | INITIAL BOILING POINT | 20% | 50% | 90% | 95% | FINAL BOILING POINT | CLOUD POINT |
| BP | 178.00 | 254.00 | 290.00 | 337.00 | 350.00 | 365.00 | -2.00 |
| TANK 5 | 180.00 | 254.00 | 290.00 | 336.00 | 349.00 | 366.00 | -2.00 |
| JET | 171.00 | 227.00 | 270.00 | 327.00 | 338.00 | 353.00 | -4.00 |
| FINA | 167.00 | 245.00 | 285.00 | 334.00 | 346.00 | 362.00 | -3.00 |
| TANK 197 | 156.00 | 212.00 | 258.00 | 325.00 | 340.00 | 360.00 | -5.00 |
| TANK 198 | 193.00 | 274.00 | 293.00 | 331.00 | 342.00 | 353.00 | -3.00 |
| Temperatures are expressed as °C. | | | | | | | |

EXAMPLE 2

The same test was carried out as in Example 1 using different fuels, but the test temperature was -15°C and the gravity test results were taken after 5 days. The results are shown in Table 3. Table 2 provides information on the fuels used.

TABLE 3

| COMPARISON OF CENTRIFUGAL AND GRAVITY WAX ANTI-SETTLING TESTS | | | |
|---|---|---|---|
| FUEL | ADDITIVE | (a) | (b) |
| BP | EVAC | 26% cloudy | 26% cloudy |
| | WASA | NWS | NWS |
| TANK 5 | EVAC | 40% cloudy | 43% cloudy |
| | WASA | NWS | NWS |
| JET | EVAC | 20% cloudy | 18% cloudy |
| | WASA | NWS | 70% cloudy |
| FINA | EVAC | 40% cloudy | 67% cloudy |
| | WASA | NWS | 98% cloudy |
| NWS = No Wax Settling (a) CENTRIFUGAL test results at -15°C after 1 hour at 500 RPM (b) GRAVITY test results at -15°C after 5 days. | | | |

Example 3

Several tests were carried out simultaneously using different amounts of WASA in each test to assess the appropriate treat level of WASA for the fuel. Each process was carried out in the same way as in example 1 and the fuel samples were centrifuged for 1 hour at 500 rpm at -15°C. The results are given in Table 4, from which it can be seen that the treat level indicated for optimum WASA performance is similar in the gravity and centrifugal tests.

4

TABLE 4

| Fuel Name: Fina | | |
|---|---|---|
| WASA Additive | gravity 5 days -15°C | centrifugal 500 rpm @ -15°C |
| ECA 11880 ppm | | |
| 0 | 67C | 40C |
| 200 | 60CL | 20CL |
| 400 | 80CL* | 10CL |
| 600 | 75CL | 8CL* |
| 800 | 65CL | 8CL |
| 1000 | 60CL | 6VCL |
| 1400 | 44VCL | NWS |

| Fuel Name: Jet | | |
|---|---|---|
| | gravity 5 days -15°C | centrifugal 500 rpm @ -15°C |
| ECA 11880 ppm | | |
| 0 | 18C | 25C |
| 200 | 20H | 10CL |
| 400 | 32H | 9CL |
| 600 | 49H | 9CL |
| 800 | 52H* | 7VCL* |
| 1000 | 51H | 6VCL |
| 1400 | 22H | 5VCL |

The results are coded as follows:

C CLEAR FUEL

H HAZY

CL CLOUDY

VCL VERY CLOUDY

NWS WAX DISPERSED (No Wax Settling)

e.g. 10VCL = 10% wax layer and 90% very cloudy fuel above

* Treat level for best WAS performance.
The fuels were treated with MDFI to give a CFPP of -15°C.

**Claims**

1. Process for assessing the amount of settling of wax in a fuel at a temperature less than the fuel cloud point comprising centrifuging the fuel at the temperature and observing the volume of settled material.

2. A process according to claim 1 in which the test temperature is 5 to 15°C below the cloud point of the fuel.

3. A process according to claim 1 or 2 in which prior to centrifuging the fuel is cooled from room temperature to the test temperature over a period of 45 minutes to 8 hours.

4. A process according to any one of the preceding claims in which the fuel is centrifuged at a speed of less than 2500 r.p.m.

5. Process according to claim 4 in which the fuel is centrifuged at from 100 to 1000 r.p.m.

6. Process according to any one of the preceding claims in which the fuel is centrifuged for 1 to 8 hours.

7. A process of assessing the amount of settling of wax in a fuel which process is substantially as hereinbefore described.

8. A process of assessing a suitable amount of wax anti-settling agent to be added to a fuel comprising carrying out on samples of the fuel a plurality of processes according to any one of the preceding claims using different amounts of wax anti-settling agent and comparing the results.